# FASCICULE DE BREVET EUROPEEN

(11) **EP 2 791 316 B1**
(45) Date de publication et mention de la délivrance du brevet: **06.01.2021**
(21) Numéro de dépôt: 12813916.9
(22) Date de dépôt: 13.12.2012
(51) Int. Cl.: C12N 1/18, C12N 1/04, B32B 27/30, B65D 77/06, B65D 85/72

(54) **MATÉRIAU ET CONDITIONNEMENT POUR LA CONSERVATION DES LEVURES**
MATERIAL UND VERPACKUNG FÜR DAS KONSERVIEREN VON HEFE
MATERIAL AND PACKAGING FOR THE CONSERVATION OF YEAST

(30) Priorité: 14.12.2011 FR 1161599
(43) Date de publication de la demande: 22.10.2014
(73) Titulaire: Lesaffre et Compagnie, 75001 Paris (FR)
(72) Inventeur: DOBEL, Sandrine, F-59830 Cysoing (FR); MALAQUIN, Anthony Bernard, F-59185 Provin (FR)
(74) Mandataire: Plasseraud IP
(86) Numéro de dépôt international: PCT/FR2012/052914
(87) Numéro de publication internationale: WO 2013/088074

(56) Documents cités:
- EP-A1- 2 019 051
- EP-A2- 1 059 163
- WO-A1-2004/012939
- WO-A1-2004/048253
- US-A- 4 863 770
- US-A- 5 726 229
- S. MARAIS ET AL: "Study of transport of small molecules through ethylene-co-vinyl acetate copolymer films. Part B: CO2 and O2 gases", POLYMER TESTING, vol. 21, 1 janvier 2002 (2002-01-01), pages 425-431, XP055029631,
- MANGARAJ S ET AL: "Applications of Plastic Films for Modified Atmosphere Packaging of Fruits and Vegetables: A Review", FOOD ENGINEERING REVIEWS, SPRINGER, no. 1, 1 janvier 2009 (2009-01-01), pages 133-158, XP002608199, ISSN: 1866-7910, DOI: 10.1007/S12393-009-9007-3 [extrait le 2009-07-15]
- -: , 27 May 2020 (2020-05-27), Retrieved from the Internet: URL:https://www.larousse.fr/dictionnaires/ francais/particulièrement/58386?q=particul ièrement#58036 [retrieved on 2020-05-27]

## Description

### Domaine de l'Invention

La présente invention se rapporte au domaine des emballages et à la conservation de produits liquides ou semi-liquides produisant du gaz, préférablement du gaz carbonique (CO₂), et concerne plus particulièrement des emballages pour le conditionnement de produits liquides ou semi-liquides contenant des levures ou des levains.

### Contexte de l'Invention

La levure en suspension aqueuse, lorsqu'elle est stockée dans des conditions favorables, présente des avantages indéniables comparée aux levures sous forme solide, dites pressées ou séchées, notamment par sa mise en œuvre simplifiée, son prédosage, ainsi que ses bonnes performances qui en font d'ailleurs un produit très apprécié des professionnels de la boulangerie. Or la levure en suspension aqueuse est un produit très sensible à ses conditions de conservation, notamment son environnement (température, pH, teneur en CO₂/O₂...), et particulièrement exposé aux contaminations. C'est ainsi un produit difficile à conditionner qui requiert donc des conditions de conservation hygiénique permettant le maintien à la fois de sa qualité microbiologique, de ses performances notamment en terme de pouvoir ferment et de ses qualités organoleptiques. En outre, l'activité et la réactivité d'une levure, si elles sont garantes de ses bonnes performances lors de son utilisation, constituent un inconvénient spécifique à la conservation d'un tel produit. Pour sa bonne conservation, il est alors souhaitable en pratique de maintenir la levure en suspension aqueuse à une température basse, de l'ordre de 4°C, de prévoir des moyens spécifiques de dégazage, notamment pour la libération des gaz issus du métabolisme de respiration de la levure, en particulier le gaz carbonique, tout en limitant les autres échanges gazeux (oxygène de l'air ambiant) notamment pour éviter le développement de contaminations.

Plusieurs solutions de conditionnement de la levure en suspension aqueuse ont été proposées. Une de ces solutions consiste à conditionner la levure liquide en Bag-in-Box. Le principe du Bag-in-Box est de disposer, dans une boîte généralement en carton (voir par exemple le brevet américain numéro US 6,223,981), une poche souple munie d'un ou de plusieurs orifices de remplissage et/ou de vidange, appelés embases (voir par exemple le brevet américain numéro US 4,863,770). Chaque embase peut être munie d'un pas-de-vis ou d'anneaux permettant, respectivement, de visser ou de clipser un bouchon. Le Bag-in-Box ainsi constitué peut être conservé, pendant plusieurs semaines, à une température comprise entre 0 et 6°C et à une humidité relative comprise entre 50 et 100%. L'utilisateur peut récupérer une quantité donnée de levure en suspension aqueuse ainsi conservée à l'aide d'une vanne ou d'un robinet fixé sur une embase après avoir retiré le bouchon. Dans le cas de la mise en place d'une vanne, l'utilisateur retourne le Bag-in-Box complètement et le place dans un dispensateur réfrigéré adapté (voir par exemple le système décrit par le présent Demandeur dans la demande internationale WO 2004/048253). Dans le cas de la mise en place d'un robinet, le Bag-in-Box est placé en position horizontale dans un réfrigérateur ou une chambre froide.

Pour éviter le gonflement de la poche souple contenue dans le carton du Bag-in-Box sous l'action du gaz carbonique produit par la levure, des orifices formant évents sont prévus comme moyens spécifiques de dégazage (voir par exemple le brevet européen EP 0 792 930-B1 et la demande internationale WO 2004/048253 du Demandeur). De plus, la poche souple est dimensionnée de manière à laisser un espace de tête suffisant dans le Bag-in-Box pour permettre au gaz d'être stocké jusqu'à ce qu'une pression suffisante permettant l'évacuation du gaz au travers du bouchon dégazeur soit atteinte. Cependant, ce système de dégazage n'est pas entièrement satisfaisant, en particulier lorsque la levure liquide conservée est une levure non-stabilisée qui produit des quantités plus importantes de CO₂ que la levure stabilisée. En effet, le gonflement de la poche souple, qui peut générer une déformation du carton lui conférant un aspect bombé, induit non seulement des problèmes de stabilité du Bag-in-Box mais peut également empêcher son insertion dans le dispensateur réfrigéré. Dans certains cas, le gonflement peut entraîner la rupture du carton. De plus, si le Bag-in-Box est sous pression, son ouverture par l'utilisateur peut produire un geyser de produit. Enfin, lors du transport ou de la manutention d'un Bag-in-Box, si de la levure entre en contact avec le bouchon dégazeur, celui-ci peut être momentanément ou définitivement colmaté.

La demande EP 2 019 051 du présent Demandeur décrit un conditionnement de produit liquide contenant de la levure comprenant un matériau perméable ayant un rapport S/M (surface d'échange S du matériau exprimée en cm² par rapport à la masse M du produit liquide contenant de la levure exprimée en gramme), et des coefficients de perméabilité à l'oxygène (O₂) et au gaz carbonique (CO₂) déterminés pour, notamment, éviter le gonflement du produit et empêcher la pénétration de contaminants. Cependant, un tel ratio S/M ne permet pas d'avoir une grande liberté sur le choix de la forme et des dimensions de la sache.

Il existe donc un besoin de disposer de conditionnements aptes à la conservation et au maintien de produits liquides contenant de la levure et permettant un meilleur dégazage du CO₂ produit par métabolisme respiratoire des levures.

### Résumé de l'Invention

D'une façon générale, la présente invention repose sur l'utilisation d'un film plastique multicouche de structure B-A-B' ayant des propriétés spécifiques de composition, d'épaisseur et de perméabilité aux gaz pour constituer la partie interne de la poche souple d'un système Bag-in-Box. Contrairement aux systèmes de dégazage existants, le dégazage obtenu par utilisation d'un film de la présente invention est constant dans le temps et uniforme sur toute la surface de la poche. Il évite donc le gonflement de la poche souple et tous les problèmes potentiels associés à ce gonflement.

Plus spécifiquement, dans un premier aspect, la présente invention concerne un film plastique tricouche comme défini dans la revendication 1 comme emballage d'un ingrédient liquide ou semi-liquide produisant du gaz, préférablement du gaz carbonique (CO₂).

Dans certains modes de réalisation, la perméabilité du film au gaz carbonique, mesurée conformément à la norme ISO 15105-2 : 2003 annexe B, est supérieure ou égale à 90 1/m².24 h à delta P = 1 bar.

La couche A consiste en un éthylène-acétate de vinyle (EVA) à haute teneur en acétate de vinyle, et chacune des couches B et B' comprend un éthylène-acétate de vinyle (EVA) ayant une teneur en acétate de vinyle inférieure à celle de l'éthylène-acétate de vinyle de la couche A. Dans certains modes de réalisation, la teneur en acétate de vinyle de l'EVA compris dans la couche B est identique à la teneur en acétate de vinyle de l'EVA compris dans la couche B'. Dans d'autres modes de réalisation, ces teneurs sont différentes. Dans certains modes de réalisation, l'EVA à haute teneur en acétate de vinyle comprend, en pourcentage massique, entre 18% et 42% d'acétate de vinyle.

Dans un deuxième aspect, la présente invention concerne un matériau plastique composé de deux films plastiques, caractérisé en ce que le premier film plastique est un film plastique trichouche de l'invention, et le second film plastique est perforé de façon homogène ou présente une perméabilité au CO₂ supérieure ou égale à celle du premier film plastique.

Dans un mode de réalisation particulier de l'invention, le second film plastique est composé de polyamide orienté (PAO) et de polyéthylène (PE) ou de polyéthylène téréphtalate (PET) et de polyéthylène (PE).

Dans un troisième aspect, la présente invention concerne un conditionnement comprenant un réservoir/récipient composé d'un matériau plastique de l'invention, caractérisé en ce que le premier film plastique du matériau définit le volume interne du réservoir/récipient.

Dans certains modes de réalisation de l'invention, le réservoir/récipient est sous forme de sache de volume interne total compris entre 1 L et 1000 L, préférablement entre 10 L et 200 L, et encore plus préférablement entre 10 L et 50 L.

La sache peut comprendre au moins une embase et un bouchon.

Dans certains modes de réalisation de l'invention, le conditionnement est sous forme de Bag-in-Box et comprend en outre une boîte en carton comprenant une ouverture. Dans certains modes de réalisation, l'embase de la sache est vissée ou clipsée dans l'ouverture du carton. Dans d'autres modes de réalisation, l'embase de la sache n'est pas attachée à l'ouverture du carton.

Dans un quatrième aspect, la présente invention concerne l'utilisation d'un matériau plastique de l'invention pour la fabrication d'un réservoir/récipient destiné à recevoir un ingrédient liquide ou semi-liquide produisant du gaz, préférablement du CO₂.

Dans un cinquième aspect, la présente invention concerne l'utilisation d'un conditionnement de l'invention pour la conservation et l'utilisation d'un ingrédient liquide ou semi-liquide produisant du gaz, préférablement du CO₂, caractérisée en ce que le conditionnement permet l'évacuation du gaz produit.

Dans un sixième aspect, la présente invention concerne un procédé de conservation et d'utilisation d'un ingrédient liquide ou semi-liquide produisant du gaz, préférablement du CO₂, comprenant les étapes suivantes :
- conditionnement de l'ingrédient produisant du gaz dans un conditionnement de l'invention,
- conservation à une température comprise entre 0 et 6°C et dans une humidité relative comprise entre 50% et 100% dudit ingrédient conditionné jusqu'à son utilisation, et
- utilisation de l'ingrédient liquide ou semi-liquide produisant du gaz.

La présente invention est telle que définie dans les revendications 1 à 15.

Dans certains modes de réalisation préférés de l'invention, l'ingrédient liquide ou semi-liquide produisant du gaz comprend un levain ou une levure, en particulier une levure liquide ou une crème de levure.

Une description plus détaillée de certains modes de réalisation préférés de l'invention est donnée ci-dessous.

### Description Détaillée de l'Invention

Comme mentionné ci-dessus, la présente invention repose sur l'utilisation d'un film plastique multicouche éventuellement renforcé par un second film plastique perforé comme emballage d'un ingrédient liquide ou semi-liquide produisant du gaz, notamment du gaz carbonique.

### I - Film Plastique Multicouche

Un film plastique multi-couche selon l'invention est caractérisé par les propriétés suivantes :
- le film a une structure de type B-A-B',
- les couches composant le film sont extrudées en une épaisseur totale comprise entre 20 et 50 microns, préférablement entre 25 et 35 microns, et plus préférablement encore, entre 27 et 32 microns,
- la perméabilité du film au gaz carbonique est supérieure ou égale à 60 1/m².24 h à delta P = 1 bar, et
- le film est faiblement imperméable à l'oxygène (O₂) et/ou à l'air et présente une perméabilité à l'oxygène inférieure ou égale à 30 1/m².24 h à delta P = 1 bar.

Le film plastique tricouche selon l'invention est généralement imperméable à l'eau liquide et de préférence à la vapeur d'eau. De plus, ce film plastique étant destiné à contenir le produit liquide ou semi-liquide contenant de la levure, il est préférablement constitué de composants appropriés aux règlementations relatives aux matériaux en contact avec les produits alimentaires.

Dans la structure tricouche B-A-B', les compositions des couches B et B' peuvent être en tout point identiques. Alternativement, les compositions des couches B et B' peuvent être différentes.

La couche A consiste en un éthylène-acétate de vinyle (EVA) à haute teneur en acétate de vinyle et chacune des couches B et B' comprend un éthylène-acétate de vinyle (EVA) à plus faible teneur en acétate de vinyle. On entend ici par « EVA à haute teneur en acétate de vinyle », un EVA contenant entre 18% et 42% d'acétate de vinyle, les pourcentages étant des pourcentages massiques. Par « EVA à plus faible teneur en acétate de vinyle », on entend ici un EVA qui contient un pourcentage plus faible en acétate de vinyle que l'EVA qui constitue la couche A. Les couches B et B' peuvent être composées du même éthylène-acétate de vinyle (contenant donc la même teneur en acétate de vinyle) ou de copolymères EVA contenant des teneurs en acétate de vinyle différentes. Dans tous les cas, ces teneurs en acétate de vinyle doivent être inférieures à celle de l'EVA qui constitue la couche A. Une couche de la structure B-A-B' peut avoir une teneur massique en acétate de vinyle constante dans la totalité de la couche. Alternativement, une couche de la structure B-A-B' peut avoir un gradient de composition, la teneur massique en acétate de vinyle étant par exemple augmentée ou abaissée dans une direction donnée de la couche.

La préparation d'un film plastique multicouche selon l'invention peut être effectuée en utilisant n'importe quelle méthode appropriée connue dans l'art. Dans certains modes de réalisation préférés, le film plastique tricouche est préparé par co-lamination or par co-extrusion. La co-extrusion peut être effectuée en utilisant une technique d'extrusion en filière plate (dite cast) ou une technique d'extrusion-soufflage. L'homme du métier saura sélectionner la technique la plus appropriée en fonction de la composition du film plastique multicouche et/ou des dimensions du film à préparer. L'homme du métier saura également déterminer les conditions opératoires de température, pression et humidité relative optimales à la mise en œuvre de la technique sélectionnée.

De manière surprenante, le présent Demandeur a constaté que, pour permettre une évacuation journalière efficace du CO₂ tout en permettant la production de poches souples sur des machines de façonnage couramment utilisées, le film plastique tricouche devait avoir une épaisseur comprise entre 20 microns et 50 microns, préférablement entre 25 microns et 35 microns, et plus préférablement encore, entre 27 microns et 32 microns.

L'utilité du film plastique multicouche selon l'invention dans l'emballage de produits liquides ou semi-liquides produisant du gaz, et en particulier de produits liquides ou semi-liquides contenant des levures, résulte de ses propriétés de perméabilité au gaz. Plus spécifiquement, un film selon l'invention présente une perméabilité au CO₂ supérieure ou égale à 80 1/m².24 h à delta P = 1 bar, préférablement supérieure ou égale à 90 1/m².24 h à delta P = 1 bar, et est faiblement imperméable à l'oxygène et/ou à l'air. Par «imperméable à l'oxygène et/ou à l'air» on entend ici un film qui présente une perméabilité à l'oxygène (O₂) qui est inférieure ou égale à 30 1/m².24 h à delta P = 1 bar.

Selon l'invention, les perméabilités au CO₂ et à l'O₂, encore appelées coefficients de perméabilité (CP), sont définies comme les coefficients de transmission respectivement du dioxyde de carbone (ou gaz carbonique) et de l'oxygène exprimées en cm³ par m² par 24h par bar (cm³/m².24h.bar ou 1/m².24h.bar) et mesurées conformément à la norme ISO 15105-2 :2003 annexe B par une méthode à détection catharométrique sur chromatographe en phase gazeuse avec une vanne d'injection et boucle d'échantillonnage. Préalablement à la mesure, le matériau est conditionné 48 heures à 23°C et à une teneur en humidité de gaz de 0%HR. La mesure du coefficient de perméabilité est effectuée à une température de 23°C, avec une humidité des gaz de 0%HR. La face externe du matériau est soumise aux gaz d'essais et les mesures effectuées sur 3 éprouvettes de 50 cm². Le gaz d'essai est constitué d'un mélange de 50% oxygène et 50% dioxyde de carbone. La détection chromatographique est effectuée à l'aide d'un détecteur Porapak(R) Q avec une température de détecteur de 140°C, un courant de filament de 200mA après étalonnage du chromatographe avec des gaz étalons à concentration connue en oxygène et dioxyde de carbone.

Pour plus de précisions dans les mesures de coefficients de faible perméabilité CP à l'O₂, (c'est-à-dire inférieur à 5000/cm³ m².24h.bar), la mesure est effectuée conformément aux normes ISO 15105-2 :2003 annexe A et ASTM D 3985-05 à l'aide d'un appareil Systech 8000. Préalablement à la mesure, le matériau est conditionné 48 heures à 23°C et une teneur en humidité de gaz de 0%HR. La mesure du coefficient de perméabilité est effectuée à une température de 23°C, avec une humidité des gaz de 0%HR. La face externe du matériau est soumise aux gaz d'essais et les mesures effectuées avec 21% d'oxygène sur 3 éprouvettes de 0.5dm². Le temps de stabilisation est de 24 heures.

Si le seuil de détection de l'appareil est atteint, il est possible de réduire la teneur en O₂ des gaz d'essai et/ou de la surface mesurée pour ainsi se placer à nouveau dans des conditions de détection. Il suffit alors de pondérer le résultat obtenu par la réduction de teneur appliquée et/ou de la réduction de surface appliquée.

Pour une mesure de coefficient de perméabilité CO₂ seulement, il est également possible d'appliquer la méthode de détection par ionisation de flammes sur chromatographe en phase gazeuse avec vanne d'injection et boucle d'échantillonnage conformément à la norme ISO 15 105-2 :2003 annexe B.

### II - Matériau Plastique

Un matériau plastique selon l'invention est composé de deux films plastiques, le premier étant un film plastique multicouche tel que décrit ci-dessus et le second film plastique étant perforé ou présentant une perméabilité au CO₂ supérieure ou égale à celle du premier film plastique. Le second film plastique a deux rôles : il confère une résistance mécanique à la poche souple ainsi réalisée, et sa perméabilité ou ses perforations permettent l'évacuation du gaz produit par la levure et évacué grâce à la perméabilité du premier film plastique.

De manière surprenante, le présent Demandeur a constaté que, lorsque le second film plastique ne présentait pas une perméabilité au CO₂ supérieure ou égale à celle du premier film plastique, seule une perforation répartie sur toute la surface du second film plastique permettait une évacuation efficace du gaz produit par les levures. Il a également été observé que, pour permettre une bonne évacuation du gaz produit par les levures conservées dans les poches souples, la densité de perforation devait être élevée. Ainsi, le second film plastique doit contenir au moins 1000 perforations/m², de préférence au moins 5000 perforations/m² et plus préférentiellement encore de l'ordre de 7000 perforations/m².

Comme indiqué ci-dessus, le second film plastique, qui est destiné à se trouver à l'extérieur de la poche souple devant contenir les levures, confère à cette poche une résistance mécanique. Le ou les composants du film plastique devront donc être choisis en conséquence. On entend ici par « résistance mécanique » toute propriété ou combinaison de propriétés de dureté, rigidité, flexibilité, élasticité, etc..., qui augmente la solidité de la poche souple. Par exemple, la résistance mécanique peut être une résistance aux chutes et/ou aux chocs. Il est, en effet, souhaitable que les réservoirs contenant les produits liquides ou semi-liquides, restent étanches lors d'un impact, sachant que lors du transport de produits industriels, des chutes occasionnelles sont quasi inévitables.

Dans certains modes de réalisation préférés, le ou les composants du second film plastique sont choisis parmi le polyéthylène (PE), le polyéthylène haute densité (PEHD), le polyéthylène basse densité (abréviation anglaise : LDPE), le polyéthylène linéaire basse densité (abréviation anglaise : LLDPE), le polycarbonate, le polyester, le polyéthylène téréphtalate (PET), le polytétrafluoroéthylène (PTFE), le polypropylène (PP), le polyamide (PA), le polyamide orienté (PAO), et leurs mélanges et/ou combinaisons. En particulier, le second film plastique peut être avantageusement composé de polyamide orienté (PAO) et de polyéthylène (PE) ou de polyéthylène téréphtalate (PET) et de polyéthylène (PE), Le second film plastique peut, par exemple, être obtenu par extrusion, co-extrusion ou lamination.

La préparation du matériau plastique selon l'invention peut se faire par n'importe quelle méthode connue dans l'art à condition que dans le matériau résultant, chacun des deux films plastiques puisse remplir son ou ses rôles (c'est-à-dire, on particulier, perméabilité au gaz carbonique et faible perméabilité à l'oxygène et/ou l'air pour le premier film plastique, résistance mécanique et évacuation du gaz grâce aux perforations ou à la perméabilité au CO₂ pour le second film plastique). Dans certains modes de réalisation préférés, le premier et le second films plastiques ne sont assemblés qu'au moment de la fabrication de la poche souple, par exemple par soudure des deux films sur les 4 côtés de la poche. Un autre exemple est le système poche dans poche, dans lequel les poches sont fixées l'une sur l'autre, soit sur les 4 côtés, soit uniquement au niveau de l'embase.

### III - Conditionnements

Un conditionnement selon l'invention généralement comprend un réservoir ou récipient constitué d'un matériau plastique comme décrit ci-dessus, dans lequel le premier film plastique définit le volume interne du réservoir qui doit contenir le produit liquide ou semi-liquide produisant du gaz. Le réservoir ou récipient peut être de n'importe quelle forme (cylindrique, cubique, parallélépipédique, plate, etc...) et/ou n'importe quelles dimensions. Dans certains modes de réalisation préférés, le réservoir en récipient est une poche souple ou sache relativement plate, en particulier une poche souple ou sache destinée à être utilisée dans un système Bag-in-Box.

Selon certains modes de réalisation, une sache selon l'invention peut contenir au moins 20 g de produit liquide ou semi-liquide produisant du gaz, par exemple entre 25 g et 600 g de produit pour des applications destinées aux consommateurs du marché grand public. Dans de tels modes de réalisation, la sache est une poche souple d'un volume interne total compris entre 20 mL et 800 mL, préférablement entre 20 mL et 500 mL,

Selon d'autres modes de réalisation, une sache selon l'invention peut contenir au moins 5 kg de produit liquide ou semi-liquide produisant du gaz, par exemple entre 10 kg et 1000 kg de produit, ou entre 10 kg et 100 kg de produit, ou encore entre 10 kg et 50 kg de produit pour des applications destinées aux professionnels, par exemple les professionnels de la boulangerie comme les artisans boulangers ou les boulangeries industrielles. Dans de tels modes de réalisation, la sache est une poche souple d'un volume interne total compris entre 1 L et 1000 L, par exemple entre 10 L et 200 L, ou entre 50 L et 500 L, ou encore entre 500 L et 1000 L.

Une sache selon l'invention peut contenir une ou plusieurs embases munies d'un pas-de-vis ou d'anneaux permettant de visser ou de clipser un bouchon. De telles embases permettent le remplissage et/ou la vidange de la sache (voir par exemple le brevet américain numéro US 4,863,770). Généralement, au moins une embase sera située en bas, lors de l'usage, de telle sorte que le produit liquide ou semi-liquide puisse être soutiré par gravité ou siphonné. La poche ou sache peut également être munie de moyens permettant une vidange ou un drainage plus aisé et/ou plus complet de la poche ou sache. De tels moyens sont notamment décrits dans DE-A-3 502 455, WO 89/00535, WO 85/0483, WO 90/06888, WO 01/79072 et EP-A-0 138 620.

Dans certains modes de réalisation, une sache selon l'invention peut permettre à l'utilisateur de déterminer ou d'estimer visuellement la quantité de produit liquide ou semi-liquide présent dans la sache. En particulier, la sache peut être transparente ou translucide, ou comporter une ou plusieurs parties transparentes ou translucides.

Dans certains modes de réalisation, un conditionnement selon l'invention est sous forme de Bag-in-Box. Dans de tels modes de réalisation, le conditionnement comprend, en plus de la poche souple ou sache, un panier grillagé porteur ou une boîte rigide (autoportant) pouvant contenir cette poche ou sache, comme décrit par exemple dans le brevet américain numéro US 6,223,981. La boîte rigide peut être un carton.

### IV - Utilisation des Conditionnements

### Ingrédients Liquides ou Semi-Liquides Produisant du Gaz

Un conditionnement selon la présente invention peut être utilisé pour la conservation de n'importe quel ingrédient liquide ou semi-liquide produisant du gaz, en particulier du CO₂. Dans certains modes de réalisation de l'invention, l'ingrédient liquide ou semi-liquide produisant du gaz comprend une levure ou un levain. Dans certains modes de réalisation particuliers de l'invention, l'ingrédient est une levure liquide ou une crème de levure.

On entend par ingrédient liquide ou semi-liquide contenant de la levure, une suspension liquide, typiquement une suspension aqueuse, comprenant de la levure. Il s'agit généralement de levure fraîche ou de levure sèche remise en suspension. Selon un mode de réalisation préférentiel de la présente invention, la levure est une levure fraîche. Avantageusement, ladite levure lors de son conditionnement comprend au moins 10⁵ unités formant des colonies (UFC) de levure par gramme, préférablement au moins 10⁸ unités formant des colonies (UFC) de levure par gramme, et avantageusement au moins 10⁹ unités formant des colonies (UFC) de levure par gramme.

L'ingrédient liquide contenant de la levure a une teneur préférentielle d'au moins 0,03% en poids de matières sèches de cellules vivantes de levure, préférablement d'au moins 0,1%, et encore plus préférablement d'au moins 5% en matières sèches de levure.

Les conditionnements selon l'invention sont particulièrement appropriés pour la conservation des levures utilisées pour leur activité fermentaire. Il s'agit notamment des levures appartenant à la famille des *Saccharomycetaceae* (classification de The Yeasts, A Taxonomic Study, Kurtzman C.P et Fell C.W., 4ème Edition, Elsevier, 1998). L'invention concerne ainsi principalement la conservation des levures de boulangerie, mais concerne aussi la conservation des levures œnologiques, de distillerie et/ou de brasserie pour lesquelles des problèmes de conservation sous forme liquide ou semi-liquide se posent.

Les levures œnologiques, de distillerie et/ou de brasserie sont préférentiellement choisies parmi le genre *Saccharomyces,* notamment *S*. *bayanus,* et *S*. *cerevisiae* en particulier les variétés *uvarum, calbergensis,* et *cerevisiae,* le genre *Kluyveromyces* en particulier *K. thermotolerans,* le genre *Brettanomyces* notamment *B. bruxellensis,* le genre *Torulaspora* en particulier *T. delbrueckii,* seules ou en mélange.

La levure de boulangerie est préférentiellement une levure choisie parmi *Saccharomyces cerevisiae, Saccharomyces chevalierii* et *Saccharomyces boulardii.*

L'ingrédient produisant du gaz (par exemple l'ingrédient contenant de la levure) est liquide ou semi-liquide, c'est-à-dire qu'il présente une viscosité inférieure à 20 000 mPa.s (centipoises), préférablement inférieure ou égale à 1 000 mPa.s (centipoises) mesurée à une température d'environ 10°C à l'aide d'un viscomètre standard par exemple un viscomètre J.P. Selecta ST2001 (L1=aiguille; vitesse=10rpm jusqu'à une viscosité de 600 mPa.s (centipoises), vitesse = 1.5rpm au-delà de 600 mPa.s (centipoises); sur un échantillon de 500 ml. Les pâtes boulangères ne sont typiquement pas des produits liquides ou semi-liquides.

L'ingrédient liquide ou semi-liquide contenant de la levure a une densité préférablement comprise entre 1,01 et 1,25, et plus préférablement encore comprise entre 1,05 et 1,15.

Par ingrédient liquide ou semi-liquide contenant de la levure, on désigne en particulier selon la présente invention, la crème de levure, préférablement de boulangerie et le levain liquide.

Par crème de levure, préférablement de boulangerie, on comprend une suspension liquide, typiquement une suspension aqueuse, de cellules vivantes de levure, préférablement de boulangerie, ladite suspension ayant une teneur préférentielle en matières sèches d'au moins 12% en masse et généralement comprise entre 12 et 50% en masse (définition étendue de crème de levure). De préférence, la crème de levure liquide ou semi-liquide répond à la définition de crème de levure au sens strict, c'est-à-dire qu'elle présente une teneur en matières sèches entre 12 et 25% en masse, et encore de préférence entre 15 et 22% en masse. Toutefois, la présente invention est également utile pour les crèmes de levure, préférablement de boulangerie, à teneur en matières sèches plus élevée, c'est-à-dire d'au moins 25% en masse, comme notamment les crèmes de levure de boulangerie dite à haute densité contenant un ou plusieurs agents osmotiques, tels que par exemple les composés polyhydroxy alimentaires et les sels alimentaires. De telles crèmes de levure de boulangerie haute densité, qui peuvent en particulier présenter une teneur en matières sèches de 25 à 48% en masse, ou encore de 25% à 46% en masse, sont connues et sont par exemple décrites dans WO 91/12315 et WO 03/048342.

Par levain liquide, on comprend selon l'invention une suspension liquide, typiquement une suspension aqueuse de cellules vivantes de levure préférablement de boulangerie, de cellules vivantes de bactéries lactiques et de farine. De préférence, le levain liquide a une teneur en matières sèches comprise entre 12 et 20% en poids, et plus préférentiellement comprise entre 15 et 17% en poids.

Des levains liquides panaires stables prêts à l'emploi et appropriés pour être conditionnés selon l'invention sont notamment ceux décrits, par le Demandeur, dans le brevet européen numéro EP 0 953 288-B1 et la demande internationale WO 2004/080187.

De manière avantageuse, le levain liquide est obtenu par la mise en œuvre d'un milieu de culture comprenant au moins une farine de céréale non-maltée et de l'eau, d'un ensemencement par au moins une préparation de bactéries lactiques hétérofermentaires et au moins une préparation de levure, préférablement par la mise en œuvre additionnelle d'au moins une farine de céréale maltée apportant des amylases ou toute source équivalente d'amylases et/ou d'au moins un ensemencement par une préparation de bactéries lactiques homofermentaires. Lors de son conditionnement, il comprend ainsi de préférence au moins 10⁶ unités fermant des colonies (UFC) de bactéries lactiques par gramme et au moins 10⁴ unités formant des colonies (UFC) de levure par gramme et encore plus préférentiellement au moins 10⁹ unités formant des colonies (UFC) de bactéries lactiques par gramme et au moins 10⁶ unités formant des colonies (UFC) de levure par gramme, présente un pH final stable compris entre 4 et 4,3 et une teneur en matière sèche entre 13 et 20% et contient préférentiellement de 15 à 30 g/kg d'acide lactique et de 6 à 10 g/kg d'acide acétique. La réalisation d'un tel levain est décrite, par exemple dans le brevet européen numéro EP 0 953 288-B1.

Un autre levain liquide panaire prêt à l'emploi particulièrement avantageux selon la présente invention comprend un milieu de culture à base de farine contenant au moins une farine de céréale et de l'eau, ledit milieu étant ensemencé et fermenté par au moins des bactéries lactiques homofermentaires biotransformatrices d'acide lactique et étant ensemencé par au moins une préparation de levures préférentiellement de boulangerie. Le levain liquide panaire prêt à l'emploi comprend préterentiellement également au moins une farine de céréale maltée apportant des amylases ou toute source équivalente d'amylases. Il comprend ainsi 10⁸ unités formant des colonies (UFC) de bactéries lactiques dont 60% sont homofermentaires biotransformatrices d'acide lactique par gramme, au moins 10⁶ unités formant des colonies (UFC) de levure par gramme, présente un pH final stable compris entre 3,8 et 4,5 et une teneur en m3atières sèches entre 27 et 35%, contient au moins 7 g d'acide acétique préférentiellement de 15 à 20 g/kg d'acide lactique et de 7 à 10 g/kg d'acide acétique.

Préférablement, les levures utilisées pour la réalisation de levain peuvent être des levures *Saccharomyces chevalierii,* et les bactéries homofermentaires sont celles de l'espèce *Lactobacillus plantarum* et/ou *casei*, les souches hétérofermentaires sont celles de l'espèce *Lactobacillus brevis*.

Dans certains modes de réalisation, le produit liquide de levure, préférablement fraîche, en particulier la crème de levure liquide et le levain liquide, est stabilisé par ajout d'un ou plusieurs stabilisants alimentaires. Ces stabilisants retardent ou évitent la décantation des cellules de levure de la suspension. Grâce à leur présence dans la suspension, le produit liquide de levure fraîche, de préférence la crème de levure ou le levain liquide conserve plus longtemps son homogénéité quand il est conservé sans agitation. Parmi les différents stabilisants alimentaires utiles pour la stabilisation de crème de levure, on peut signaler les gommes, telles que la gomme de xanthane, et des amidons thermiquement et/ou chimiquement modifiés, tels que l'adipate de diamidon acétylé répondant à la définition de l'amidon modifié E1422. De telles crèmes de levure stabilisées sont par exemple décrites dans EP-A-0 792 930.

Les compositions de levure ou de levain peuvent aussi contenir des additifs ou auxiliaires ayant un rôle d'améliorant de panification et/ou un rôle de maintien de l'homogénéité de la suspension. Ces additifs peuvent être des oxydants comme l'acide ascorbique, des réducteurs comme la L-cystéine, des préparations enzymatiques présentant une ou plusieurs activités enzymatiques comme des préparations d'amylase, de xylanase, de lipase et/ou phospholipidase, oxydase comme la glucose oxydase. Ces additifs peuvent aussi être un ou plusieurs agents osmotiques, tels que par exemple les composés polyhydroxy alimentaires et les sels alimentaires.

### Conservation d'Ingrédients Liquides ou Semi-Liquides Produisant du Gaz

La présente invention a également pour objet l'utilisation d'un conditionnement tel que décrit ci-dessus pour conserver un ingrédient liquide ou semi-liquide produisant du gaz, notamment un ingrédient liquide ou semi-liquide contenant de la levure comme décrit ci-dessus. Un tel conditionnement est plus particulièrement utilisé à des températures inférieures à 8°C, préférablement entre 0°C et 6°C, une humidité relative comprise entre 50% et 100%, et permet une bonne conservation des produits liquides ou semi-liquides contenant des levures pendant au moins 4 semaines, préférablement pendant au moins 6 semaines, et plus préférablement encore pendant au moins 8 semaines.

Dans ces conditions de conservation, un tel conditionnement peut, en outre, être utilisé avec une variation potentielle de température pouvant aller jusqu'à 35°C pendant une période maximale de 8 heures, et préférablement pendant 4 heures, et encore plus préférablement pouvant aller jusqu'à 20°C pendant une période maximale de 2 heures.

A moins qu'ils ne soient définis d'une autre manière, tous les termes techniques et scientifiques utilisés dans la description ont la même signification que celle couramment comprise par un spécialiste ordinaire du domaine auquel appartient cette invention. De même, toutes les publications, demandes de brevet, tous les brevets et toutes autres références mentionnés ici sont incorporés par référence.

### Exemples

Les exemples suivants décrivent certains modes de réalisation de la présente invention. Cependant, il est entendu que les exemples ne sont présentés qu'à titre illustratif seulement et ne limitent en aucun cas la portée de l'invention.

### Exemple 1

Des films tricouches à base d'Ethylène Acétate de Vinyle (EVA) ont été préparés par les techniques d'extrusion gonflage bien connues de l'homme du métier. Deux films, film 1 et film 2, ont été obtenus d'épaisseur 30 µm et 40 µm, respectivement. La couche centrale A de chacun de ces films consiste en un EVA ayant une teneur massique en acétate de vinyle entre 18% et 42% et les couches externes B et B' sont en EVA ayant une teneur massique en acétate de vinyle inférieure à celle de l'EVA de la couche A.

***Mesures de perméabilité.*** Les coefficients de transmission de l'oxygène et du gaz carbonique ont été déterminés sur un échantillon de film dans des conditions standard et après conditionnement en enceinte climatique à 23°C et 90% d'humidité relative (HR) pendant 48 heures.

Les coefficients de transmission de l'oxygène et du gaz carbonique ont été déterminés sur un appareillage LYSSY GPM5000. Chaque échantillon a été placé dans une cellule de test de 50 cm² entre deux chambres, la chambre inférieure étant constamment balayée par un flux d'hélium et l'autre chambre étant en contact avec le gaz test (un mélange CO₂/O₂/N₂ dans les proportions 1/3, 1/3, 1/3). Tout gaz qui traverse l'échantillon de film est transporté par le gaz vecteur jusqu'au chromatographe en phase gazeuse muni d'un détecteur catharomètre. Les conditions expérimentales utilisées sont les suivantes :
- gaz vecteur : hélium,
- gaz test : mélange dans les proportions 1/3, 1/3, 1/3 de CO₂/O₂/N₂,
- température de l'essai : 23°C,
- les films sont testés en l'état puis après conditionnement à 23°C et 90% HR pendant 48 heures.

L'épaisseur de l'échantillon de film a été mesurée à l'aide d'un micromètre de précision ADAMEL LHOMARGIE (précision 1 µm). Les mesures ont été effectuées sur trois échantillons différents de chaque film.

***Résultats.*** Les résultats obtenus sont présentés dans les Tableaux 1 et 2 suivants, dans lesquels, PCO₂ et PO₂ sont exprimés en 1/m²/24h/atm.

**Tableau 1. Epaisseur et coefficients de transmission de l'oxygène et du carbonique pour trois échantillons du film 1.**

| **Film 1** | | **Epaisseur (µm)** | **PCO₂** | **PO₂** |
|---|---|---|---|---|
| Conditions standard | Echantillon 1 | 28 | 87 | 14 |
| | Echantillon 2 | 27 | 87 | 21 |
| | Echantillon 3 | 26 | 93 | 18 |
| Après conditionnement | Echantillon 1 | 25 | 101 | 19 |
| | Echantillon 2 | 28 | 111 | 23 |
| | Echantillon 3 | 20 | 123 | 26 |

**Tableau 2. Epaisseur et coefficients de transmission de l'oxygène et du carbonique pour trois échantillons du film 2.**

| **Film 2** | | **Epaisseur (µm)** | **PCO₂** | **PO₂** |
|---|---|---|---|---|
| Conditions standard | Echantillon 1 | 36 | 66 | 12 |
| | Echantillon 2 | 37 | 80 | 15 |
| | Echantillon 3 | 34 | 87 | 16 |
| Après conditionnement | Echantillon 1 | 39 | 78 | 14 |
| | Echantillon 2 | 37 | 91 | 18 |
| | Echantillon 3 | 39 | 88 | 19 |

Les perméabilités des films avant et après conditionnement ne diffèrent pas de manière significative.

### Exemple 2

A partir des films de l'Exemple 1, des saches de 10 et 20 kg, destinées à l'emballage de levure liquide et/ou de crème de levure, ont été préparés. Les saches vides ont une forme rectangulaire ou carrée de dimensions intérieures 500x680 pour les saches de 20 kg et 500x500 pour les saches de 10 kg.

Les saches ont été renforcées par un second film multicouche ayant la composition suivante : une couche de 20 µm d'OPA (Nylon) de densité 23 g/m², une couche d'adhésif, et une couche de 80 µm LLDPE/LDPE de densité de 74 g/m².

Le second film est perforé, comprenant 7090 perforations par m². Les saches ont été munies d'une embase à visser positionnée à 105 mm du scellage supérieur.

Des saches témoins ont été utilisées comme contrôles. Ces saches témoins sont constituées d'un film de polyéthylène faiblement perméable au CO₂ (perméabilité de 17 à 27 l/m²/24h à delta P=lbar), et munies d'un bouchon dégazeur permettant l'évacuation du gaz carbonique dégagé au cours du stockage et du transport.

***Test de mesure de dégagement gazeux.*** L'objectif de ce test était d'évaluer la quantité de CO₂ s'évacuant, au cours du stockage, au travers d'un Bag-in-Box (BIB) selon l'invention (c'est-à-dire « carton + sache selon l'invention ») et au travers d'un BIB témoin (c'est-à-dire « carton + sache témoin ») après que chacun ait été rempli d'un produit liquide contenant de la levure issu d'une même production. Pour cela, chaque BIB rempli a été placé dans son intégralité dans une poche étanche. Ainsi, tout gaz s'évacuant du système d'emballage se retrouve piégé dans la poche. Chaque jour, ce gaz est vidé de la poche étanche en étant mesuré grâce au principe de l'éprouvette : le système, similaire à une éprouvette, est tout d'abord rempli d'eau. On ouvre alors le robinet de la sache contenant le BIB et de l'éprouvette. Le gaz chasse l'eau et on mesure le volume de gaz ainsi évacué. Les résultats obtenus sont reportés dans le Tableau 3 suivant.

**Tableau 3. Quantité de CO₂ qui s'évacue des différents BIB testés en fonction du temps de stockage.**

| **Temps de stockage (jours)** | **BIB 10 kg** | | **BIB 20 kg** | |
|---|---|---|---|---|
| | **selon l'invention (ml)** | **témoin (ml)** | **selon l'invention (ml)** | **témoin (ml)** |
| 1 | 1860 | 1220 | 2220 | 2100 |
| 2 | | | | |
| 3 | 2320 | 2480 | 4320 | 4400 |
| 4 | | | | |
| 5 | | | | |
| 6 | 3500 | 3680 | 7120 | 7200 |
| 7 | 1100 | 1040 | 2300 | 2300 |
| 8 | 1080 | 1140 | 1280 | 1260 |
| 9 | 360 | 800 | 1460 | 1320 |
| 10 | 1080 | 1180 | 2300 | 2200 |
| 11 | | | | |
| 12 | | | | |
| 13 | 2580 | 2840 | 4940 | 5340 |
| 14 | 890 | 1600 | 1120 | 1290 |
| 15 | 900 | 1140 | 1300 | 1440 |
| 16 | | | | |
| 17 | 1980 | 1960 | 3560 | 4200 |
| 18 | | | | |
| 19 | | | | |
| 20 | 3060 | 3180 | 5200 | 5400 |
| 21 | 980 | 920 | 1260 | 1320 |
| 22 | 1200 | 1200 | 2080 | 2180 |
| 23 | 1000 | 980 | 1260 | 1500 |
| 24 | 1080 | 980 | 2080 | 1560 |
| | | | | |
| **Total (ml)** | **24970** | **26340** | **43800** | **45010** |
| **Moyenne par jour (litre)** | **1,04** | **1,10** | **1,83** | **1,88** |

Comme on peut le constater dans le Tableau 3, le comportement des BIB selon l'invention et des BIB témoins est identique, qu'il s'agisse de la quantité moyenne de gaz évacué par jour, ou du volume total de CO₂ évacué au cours des 24 jours d'essais (comme le montre le Tableau 4).

**Tableau 4. Quantité totale de CO₂ évacués des différents BIB en 24 jours de stockage.**

| | **BIB 10 kg** | | **BIB 20 kg** | |
|---|---|---|---|---|
| | **selon l'invention (ml)** | **témoin (ml)** | **selon l'invention (ml)** | **témoin (ml)** |
| **Volume total de CO₂ évacué en 24 jours (litre)** | 25 | 27 | 44 | 45 |

## Revendications

1. Utilisation d'un film plastique tricouche de structure B-A-B' comme emballage d'un ingrédient liquide ou semi-liquide produisant du gaz, ledit ingrédient liquide ou semi-liquide comprenant un levain ou une levure et présentant une viscosité inférieure à 20 000 mPa.s (centipoises), préférablement inférieure ou égale à 1 000 mPa.s (centipoises) mesurée à une température d'environ 10°C à l'aide d'un viscomètre tel que défini dans la description, **caractérisé en ce que** :
- la couche A consiste en un éthylène-acétate de vinyle (EVA) à teneur en acétate de vinyle, en pourcentage massique, compris entre 18% et 42%, et
- chacune des couches B et B' consiste en un éthylène-acétate de vinyle (EVA) ayant une teneur en acétate de vinyle inférieure à celle de l'éthylène-acétate de vinyle de la couche A,
et **caractérisé en ce que** :
- la perméabilité du film au gaz carbonique, mesurée conformément à la norme ISO 15105-2 : 2003 annexe B, est supérieure ou égale à 80 l/m².24 h à delta P = 1 bar,
- le film a une épaisseur totale de 20 à 50 microns, de préférence de 30 microns, et
- la perméabilité du film à l'oxygène (O₂), mesurée conformément à la norme ISO 15105-2 : 2003 annexe B, est inférieure ou égale à 30 l/m².24 h à delta P = 1 bar.

2. Utilisation selon la revendication 1, **caractérisée en ce que** la perméabilité du film au gaz carbonique, mesurée conformément à la norme ISO 15105-2 : 2003 annexe B, est supérieure ou égale à 90 l/m².24 h à delta P = 1 bar.

3. Utilisation selon la revendication 1 ou la revendication 2, **caractérisée en ce que** les couches B et B' sont identiques.

4. Utilisation selon la revendication 1 ou la revendication 2, **caractérisée en ce que** les couches B et B' sont différentes.

5. Matériau plastique composé de deux films plastiques, **caractérisé en ce que** le premier film plastique est un film plastique tricouche comme défini dans l'une quelconque des revendications 1 à 4, et le second film plastique est perforé de façon homogène ou présente une perméabilité au gaz carbonique supérieure ou égale à celle du premier film plastique.

6. Matériau plastique selon la revendication 5, **caractérisé en ce que** le second film plastique, lorsqu'il est perforé, contient au moins 1000 perforations/m², de préférence au moins 5000 perforations/m² et plus préférentiellement encore de l'ordre de 7000 perforations/m².

7. Matériau plastique selon la revendication 5 ou la revendication 6, **caractérisé en ce que** le second film plastique est composé de polyamide orienté (PAO) et de polyéthylène (PE) ou de polyéthylène téréphtalate (PET) et de polyéthylène (PE).

8. Conditionnement comprenant un réservoir/récipient composé d'un matériau plastique selon l'une quelconque des revendications 5 à 7, **caractérisé en ce que** le premier film plastique du matériau définit le volume interne du réservoir/récipient.

9. Conditionnement selon la revendication 8, **caractérisé en ce que** le réservoir/récipient est sous forme de sache de volume interne total compris entre 1 L et 1000 L, préférablement entre 10 L et 200 L, et plus préférablement entre 1 L et 50 L.

10. Conditionnement selon la revendication 9, **caractérisé en ce que** la sache comprend une embase et un bouchon.

11. Conditionnement selon la revendication 10, **caractérisé en ce que** la conditionnement est sous forme de Bag-in-Box et comprend en outre une boîte en carton comprenant une ouverture.

12. Utilisation d'un matériau plastique selon l'une quelconque des revendications 5 à 7 pour la fabrication d'un récipient/réservoir destiné à recevoir un ingrédient liquide ou semi-liquide produisant du gaz, préférablement du gaz carbonique, ledit ingrédient liquide ou semi-liquide comprenant un levain ou une levure et présentant une viscosité inférieure à 20 000 mPa.s (centipoises), préférablement inférieure ou égale à 1 000 mPa.s (centipoises) mesurée à une température d'environ 10°C à l'aide d'un viscomètre tel que défini dans la description.

13. Utilisation d'un conditionnement selon l'une quelconque des revendications 8 à 11 pour la conservation et l'utilisation d'un ingrédient liquide ou semi-liquide produisant du gaz, préférablement du gaz carbonique, ledit ingrédient liquide ou semi-liquide comprenant un levain ou une levure et présentant une viscosité inférieure à 20 000 mPa.s (centipoises), préférablement inférieure ou égale à 1 000 mPa.s (centipoises) mesurée à une température d'environ 10°C à l'aide d'un viscomètre tel que défini dans la description, **caractérisée en ce que** le conditionnement permet l'évacuation du gaz produit.

14. Procédé de conservation et d'utilisation d'un ingrédient liquide ou semi-liquide produisant du gaz, préférablement du gaz carbonique, ledit ingrédient liquide ou seli-liquide comprenant un levain ou une levure et présentant une viscosité inférieure à 20 000 mPa.s (centipoises), préférablement inférieure ou égale à 1 000 mPa.s (centipoises) mesurée à une température d'environ 10°C à l'aide d'un viscomètre tel que défini dans la description, comprenant les étapes suivantes :
- conditionnement de l'ingrédient produisant du gaz dans un conditionnement selon l'une quelconque des revendications 8 à 11,
- conservation à une température comprise entre 0 et 6°C et dans une humidité relative comprise entre 50% et 100% dudit ingrédient conditionné jusqu'à son utilisation, et
- utilisation de l'ingrédient liquide ou semi-liquide produisant du gaz.

15. Utilisation selon l'une quelconque des revendications 1 à 4, 12 et 13 ou procédé selon la revendication 14, **caractérisé en ce que** l'ingrédient liquide ou semi-liquide produisant du gaz est une levure liquide ou une crème de levure.

## Patentansprüche

1. Verwendung eines dreischichtigen Kunststofffilms der Struktur B-A-B' als Verpackung für einen flüssigen oder halbflüssigen Bestandteil, der Gas erzeugt, wobei der flüssige oder halbflüssige Bestandteil einen Sauerteig oder eine Hefe umfasst und eine Viskosität von weniger als 20 000 mPa.s (Centipoise) aufweist, bevorzugt weniger als oder gleich 1 000 mPa.s (Centipoise), gemessen bei einer Temperatur von etwa 10 °C mit Hilfe eines Viskosimeters wie in der Beschreibung definiert, **dadurch gekennzeichnet dass**:
- die Schicht A aus einem Ethylen-Vinylacetat (EVA) mit einem Vinylacetatgehalt, in Massenprozent, zwischen 18 % und 42 % besteht und
- jede der Schichten B und B' aus einem Ethylen-Vinylacetat (EVA) besteht, das einen geringeren Vinylacetatgehalt aufweist als der des Ethylen-Vinylacetats der Schicht A
und **dadurch gekennzeichnet, dass**:
- die Permeabilität des Films für Kohlenstoffdioxidgas, gemessen gemäß der ISO Norm 15105-2: 2003 Anhang B, größer als oder gleich 80 l/m².24 h bei delta P = 1 bar ist,
- der Film eine Gesamtdicke von 20 bis 50 Mikrometer, vorzugsweise 30 Mikrometer aufweist, und
- die Permeabilität des Films für Sauerstoff (O₂), gemessen gemäß der ISO Norm 15105-2: 2003 Anhang B, weniger als oder gleich 30 l/m².24 h bei delta P = 1 bar ist.

2. Verwendung nach Anspruch 1, **dadurch gekennzeichnet, dass** die Permeabilität des Films für Kohlenstoffdioxidgas, gemessen gemäß der ISO Norm 15105-2: 2003 Anhang B, größer als oder gleich 90 l/m².24 h bei delta P = 1 bar ist.

3. Verwendung nach Anspruch 1 oder Anspruch 2, **dadurch gekennzeichnet, dass** die Schichten B und B' identisch sind.

4. Verwendung nach Anspruch 1 oder Anspruch 2, **dadurch gekennzeichnet, dass** die Schichten B und B' unterschiedlich sind.

5. Kunststoffmaterial, das aus zwei Kunststofffilmen zusammengesetzt ist, **dadurch gekennzeichnet, dass** der erste Kunststofffilm ein dreischichtiger Kunststofffilm gemäß einem der Ansprüche 1 bis 4 ist und der zweite Kunststofffilm gleichmäßig perforiert ist oder eine Permeabilität für Kohlenstoffdioxidgas größer als oder gleich der des ersten Kunststofffilms aufweist.

6. Kunststoffmaterial nach Anspruch 5, **dadurch gekennzeichnet, dass** der zweite Kunststofffilm, wenn er perforiert ist, mindestens 1000 Perforationen/m² enthält, bevorzugt mindestens 5000 Perforationen/m² und stärker bevorzugt noch etwa 7000 Perforationen/m².

7. Kunststoffmaterial nach Anspruch 5 oder Anspruch 6, **dadurch gekennzeichnet, dass** der zweite Kunststofffilm aus orientiertem Polyamid (PAO) und Polyethylen (PE) oder aus Polyethylenterephthalat (PET) und Polyethylen (PE) zusammengesetzt ist.

8. Verpackungsmaterial, umfassend ein Reservoir/einen Behälter, das/der aus einem Kunststoffmaterial nach einem der Ansprüche 5 bis 7 zusammengesetzt ist, **dadurch gekennzeichnet, dass** der erste Kunststofffilm des Materials das Innenvolumen des Reservoirs/Behälters definiert.

9. Verpackungsmaterial nach Anspruch 8, **dadurch gekennzeichnet, dass** das Reservoir/der Behälter in Form eines Beutels mit einem Gesamtinnenvolumen zwischen 1 l und 1000 l, vorzugsweise zwischen 10 l und 200 l und bevorzugter zwischen 1 l und 50 l vorliegt.

10. Verpackungsmaterial nach Anspruch 9, **dadurch gekennzeichnet, dass** der Beutel einen Boden und einen Stopfen umfasst.

11. Verpackungsmaterial nach Anspruch 10, **dadurch gekennzeichnet, dass** das Verpackungsmaterial in Form einer Bag-in-Box vorliegt und ferner eine Kartonschachtel umfasst, die eine Öffnung umfasst.

12. Verwendung eines Kunststoffmaterials nach einem der Ansprüche 5 bis 7 zur Herstellung eines Behälters/Reservoirs, der/das dazu geeignet ist einen flüssigen oder halbflüssigen Bestandteil, der Gas erzeugt, vorzugsweise Kohlenstoffdioxidgas, aufzunehmen, wobei der flüssige oder halbflüssige Bestandteil einen Sauerteig oder eine Hefe umfasst und eine Viskosität von weniger als 20 000 mPa.s (Centipoise) aufweist, bevorzugt weniger als oder gleich 1 000 mPa.s (Centipoise), gemessen bei einer Temperatur von etwa 10 °C mit Hilfe eines Viskosimeters wie in der Beschreibung definiert.

13. Verwendung eines Verpackungsmaterials nach einem der Ansprüche 8 bis 11 zur Aufbewahrung und Verwendung eines flüssigen oder halbflüssigen Bestandteils, der Gas erzeugt, vorzugsweise Kohlenstoffdioxidgas, wobei der flüssige oder halbflüssige Bestandteil einen Sauerteig oder eine Hefe umfasst und eine Viskosität von weniger als 20 000 mPa.s (Centipoise) aufweist, bevorzugt weniger als oder gleich 1 000 mPa.s (Centipoise), gemessen bei einer Temperatur von etwa 10 °C mit Hilfe eines Viskosimeters wie in der Beschreibung definiert, **dadurch gekennzeichnet, dass** das Verpackungsmaterial die Evakuierung des produzierten Gases ermöglicht.

14. Verfahren zur Aufbewahrung und Verwenden eines flüssigen oder halbflüssigen Bestandteils, der Gas erzeugt, vorzugsweise Kohlenstoffdioxidgas, wobei der flüssige oder halbflüssige Bestandteil einen Sauerteig oder eine Hefe umfasst und eine Viskosität von weniger als 20 000 mPa.s (Centipoise) aufweist, bevorzugt weniger als oder gleich 1 000 mPa.s (Centipoise), gemessen bei einer Temperatur von etwa 10 °C mit Hilfe eines Viskosimeters wie in der Beschreibung definiert, umfassend die folgenden Schritte:
- Verpacken des Bestandteils, der Gas erzeugt, in einem Verpackungsmaterial gemäß einem der Ansprüche 8 bis 11,
- Lagerung bei einer Temperatur zwischen 0 und 6 °C und bei einer relativen Feuchtigkeit zwischen 50% und 100% des verpackten Bestandteils bis zu seiner Verwendung und
- Verwendung des flüssigen oder halbflüssigen Bestandteils, der Gas erzeugt.

15. Verwendung nach einem der Ansprüche 1 bis 4, 12 und 13 oder Verfahren nach Anspruch 14, **dadurch gekennzeichnet, dass** der flüssige oder halbflüssige Bestandteil, der Gas erzeugt, eine flüssige Hefe oder eine Hefecreme ist.

## Claims

1. Use of a three-layer plastic film with a B-A-B' structure as wrapping for a liquid or semi-liquid ingredient that produces gas, said liquid or semi-liquid ingredient comprising a leaven or a yeast and having a viscosity of less than 20 000 mPa.s (centipoises), preferably less than or equal to 1 000 mPa.s (centipoises) measured at a temperature of approximately 10°C using a standard viscometer such as described in the specification, **characterized in that**:
- layer A consists of an ethylene-vinyl acetate (EVA) with a vinyl acetate content, as percentage by weight, between 18% and 42%, and
- each of layers B and B' consists of an ethylene-vinyl acetate (EVA) having an ethylene-vinyl acetate content which is less than that of the ethylene-vinyl acetate of layer A,
and **characterized in that**:
- the permeability of the film to carbon dioxide, measured according to ISO standard 15105-2:2003 annex B, is greater than or equal to 80 l/m².24 h at delta P = 1 bar,
- the film has a total thickness of from 20 to 50 microns, preferably of 30 microns, and
- the permeability of the film to oxygen (O₂), measured according to ISO standard 15105-2:2003 annex B, is less than or equal to 30 l/m².24 h at delta P = 1 bar.

2. The use according to claim 1, **characterized in that** the permeability of the film to carbon dioxide, measured according to ISO standard 15105-2:2003 annex B, is greater than or equal to 90 l/m².24 h at delta P = 1 bar.

3. The use according to claim 1 or claim 2, **characterized in that** layers B and B' are identical.

4. The use according to claim 1 or claim 2, **characterized in that** layers B and B' are different.

5. A plastic material composed of two plastic films, **characterized in that** the first plastic film is a three-layer plastic film as defined in any one of claims 1 to 4, and the second plastic film is uniformly perforated or has a permeability to carbon dioxide which is greater than or equal to that of the first plastic film.

6. The plastic material according to claim 5, **characterized in that** the second plastic film, when perforated, contains at least 1000 perforations/m², preferably at least 5000 perforations/m² and even more preferentially about 7000 perforations/m².

7. The plastic material according to claim 5 or claim 6, **characterized in that** the second plastic film is composed of oriented polyamide (OPA) and polyethylene (PE) or of polyethylene terephthalate (PET) and polyethylene (PE).

8. A packaging comprising a reservoir/container composed of a plastic material according to any one of claims 5 to 7, **characterized in that** the first plastic film of the plastic material defines the internal volume of the reservoir/container.

9. The packaging according to claim 8, **characterized in that** the reservoir/container is in the form of a pouch having a total internal volume of between 1 l and 1000 l, preferably between 10 l and 200 l, and more preferably between 1 l and 50 l.

10. The packaging according to claim 9, **characterized in that** the pouch comprises a base and a cap.

11. The packaging according to claim 10, **characterized in that** the packaging is in the form of a Bag-in-Box and also comprises a cardboard box comprising an opening.

12. Use of a plastic material according to any one of claims 5 to 7, for manufacturing a container/reservoir intended to receive a liquid or semi-liquid ingredient which produces gas, preferably carbon dioxide, said liquid or semi-liquid ingredient comprising a leaven or a yeast and having a viscosity of less than 20 000 mPa.s (centipoises), preferably less than or equal to 1 000 mPa.s (centipoises) measured at a temperature of approximately 10°C using a standard viscometer such as described in the specification.

13. Use of a packaging according to any one of claims 8 to 11 for the storage and use of a liquid or semi-liquid ingredient which produces gas, preferably carbon dioxide, said liquid or semi-liquid ingredient comprising a leaven or a yeast and having a viscosity of less than 20 000 mPa.s (centipoises), preferably less than or equal to 1 000 mPa.s (centipoises) measured at a temperature of approximately 10°C using a standard viscometer such as described in the specification, **characterized in that** the packaging allows evacuation of the gas produced.

14. Method for the storage and use of a liquid or semi-liquid ingredient which produces gas, preferably carbon dioxide, said liquid or semi-liquid ingredient comprising a leaven or a yeast and having a viscosity of less than 20 000 mPa.s (centipoises), preferably less than or equal to 1 000 mPa.s (centipoises) measured at a temperature of approximately 10°C using a standard viscometer such as described in the specification, comprising the following steps:
- packaging of the ingredient which produces gas in a packaging according to any one of claims 8 to 11,
- storage, at a temperature of between 0 and 6°C and in a relative humidity of between 50% and 100%, of said packaged ingredient until use, and
- use of the liquid or semi-liquid ingredient which produces gas.

15. The use according to any one of claims 1 to 4, 12 and 13 or the method according to claim 14, **characterized in that** the liquid or semi-liquid ingredient which produces gas is a liquid yeast or a cream yeast.
